# EUROPEAN PATENT SPECIFICATION

(11) **EP 1 868 993 B1**
(45) Date of publication and mention of the grant of the patent: **30.04.2014**
(21) Application number: 06727218.7
(22) Date of filing: 07.04.2006
(51) Int. Cl.: C07D 207/34, A61K 31/40, A61P 3/06

(54) **PROCESS FOR THE PREPARATION OF A NEW CRYSTALLINE ATORVASTATIN HEMICALCIUM SALT POLYMORPH FORM**
VERFAHREN ZUR HERSTELLUNG EINER NEUEN KRISTALLINEN POLYMORPHFORM VON ATORVASTATIN-HEMICALCIUMSALZ
PROCEDE POUR LA PREPARATION D'UNE NOUVELLE FORME POLYMORPHE DE SEL D'HEMICALCIUM D'ATORVASTATINE CRISTALLIN

(30) Priority: 08.04.2005 HU 0500370; 14.02.2006 HU 0600120
(43) Date of publication of application: 26.12.2007
(73) Proprietor: EGIS Gyógyszergyár Nyilvánosan Müködõ Részvénytársaság, 1106 Budapest (HU)
(72) Inventor: BARKÓCZY, Jozsef, H-1016 Budapest (HU); KÓTAY NAGY, Péter, H-2600 Vác (HU); SIMIG, Gyula, H-1126 Budapest (HU); SZENT-KIRÁLLYI, Zsuzsanna, H-1223 Budapest (HU); BARTHA, Ferenc, H-4440 Tiszavasvári (HU); KATONA, Zoltán, H-3300 Eger (HU); VERECZKEYN DONÁTH, Gyorgyi, H-1034 Budapest (HU); NAGY, Kálmán, H-1025 Budapest (HU); NEMETH, Norbert, H-9400 Sopron (HU); RUZSICS, György, H-7191 Hogyész (HU); CSELENYAK, Judit, H-1124 Budapest (HU)
(74) Representative: Hirsch & Associés
(86) International application number: PCT/HU2006/000026
(87) International publication number: WO 2006/106372

(56) References cited:
- WO-A-02/43732

## Description

### FIELD OF THE INVENTION

The present invention relates to a process for the preparation of a new crystalline polymorph form of atorvastatin hemicalcium salt [(3R, 5R)-7-[3-phenyl-4-[(phenylcarbamoyl]-2-(4-fluorophenyl)-5-(1-methylethyl)-1H-pyrrole-1-yl]-3,5-dihydroxy-heptanoic acid calcium salt (2:1)] of the Formula (I),

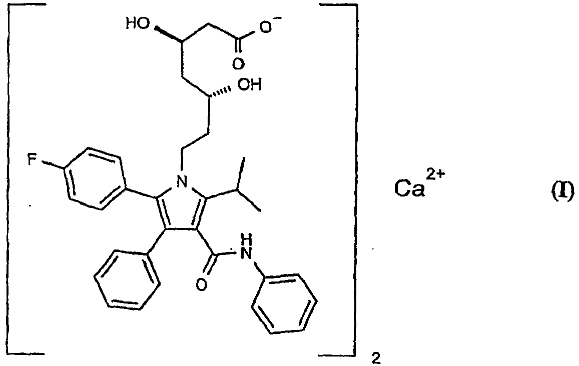

It is also disclosed medicinal preparations containing the new polymorph form, the use of the new polymorph form for the preparation of medicinal products.

### TECHNICAL BACKGROUND OF THE INVENTION

Atorvastatin hemicalcium salt [(3R, 5R)-7-[3-phenyl-4-[(phenylcarbamoyl]-2-(4-fluorophenyl)-5-(1-methylethyl)-1H-pyrrole-1-yl]-3,5-dihydroxy-heptanoic acid calcium salt (2:1)] of the Formula (I), which is designated in the subsequent part of the present application as atorvastatin hemicalcium salt, is an inhibitor of the enzyme 3-hydroxy-3-methyl-glutaryl-coenzime A reductase present in the liver. Said enzyme plays distinguished role in the biosynthesis of cholesterol. Due to this effect, atorvastatin hemicalcium salt of the Formula (I) can be very effectively used as pharmaceutical active ingredient for decreasing blood lipid- and cholesterol concentration.

Several processes for the preparation of atorvastatin hemicalcium salt have been disclosed. Said processes comprise the subject matter of European Patents No. 330172, 409281, 448552, 553213 and 687263. In the subsequent part of the present application, atorvastatin hamicalcium salt prepared according to European Patent No. 409281 is referred as crude atorvastatin hemicalcium salt.

Processes for the preparation of crystalline polymorph form I, II and IV of atorvastatin hemicalcium salt have been disclosed in European Patent No. 848705. According to said patent, polymorph I crystalline form of atorvastatin hemicalcium salt is obtained either by seeding the solution of atorvastatin hemicalcium salt with seed crystals of polymorph I form atorvastatin hemicalcium salt or by suspending and stirring the mixture of amorphous and crystalline form I atorvastatin hemicalcium salt in 200-fold volume of methanol-water mixture at 40 °C and crystallizing polymorph form I atorvastatin hemicalcium salt during the period of 17 hours.

Crystalline polymorph form II atorvastatin hemicalcium salt is prepared from the mixture of amorphous atorvastatin hemicalcium salt and crystalline polymorph form I atorvastatin hemicalcium salt by suspending the starting material in 20-fold amount of 3:2(v/v) mixture of methanol and water and stirring the resulting suspension for 3 days.

Crystalline polymorph form IV atorvastatin hemicalcium can be prepared from atorvastatin lactone. The lacton is converted into hemicalcium salt in solution, the aqueous mixture is heated at least to 65-70 °C for 5 minutes, and cooled to 55-65 °C. The crystals are filtered, stirred in methanol at 55-65 °C, the suspension is cooled to 25-30 °C and the polymorph form IV of atorvastatin hemicalcium is filtered off.

Crystalline atorvastatin hemicalcium polymorph form III has been disclosed in European Patent No. 848704.

Beyond the above mentioned crystalline forms, numerous further crystalline polymorph forms of atorvastatin hemicalcium salt have been prepared. Such crystalline forms are disclosed in European Patent Application Nos. 1235799, 1332130, 1341785, 1363621, 1425287, 1465865, 1480950, 1423364, 1414796, 1472220 and in the International Patent Application No. WO 02/43732. Crystalline forms of atorvastatin hemicalcium salt of the Formula (I) additionally to atorvastatin hemicalcium contain 1-9 mol water bound in the crystalline lattice, calculated calculated on the basis of the molar amount of atorvastatin hemicalcium salt.

According to the state of the art, the amorphous atorvastatin hemicalcium salt is also known and can be prepared by the processes disclosed in European Patent No. 839132 or in International Patent Application No. WO 01/28999.

The common feature of all the above mentioned processes directed to the preparation of crystalline forms of atorvastatin hemicalcium salt resides in the fact that said processes utilize controlled crystallization, wherein the polarity of the solution containing atorvastatin hemicalcium salt is decreased by the addition of apolar or less polar solvent, until atorvastatin hemicalcium is obtained in crystalline form, or solid amorphous or crystalline atorvastatin hemicalcium salt is stirred-in a solvent or a solvent mixture until a crystalline form of atorvastatin hemicalcium salt different from that of the starting material is obtained.

In the conventional crystallization process, however, solvent is added to the substance to be recrystallized and the resulting suspension is heated until dissolution. The solution thus obtained is filtered to remove mechanical and less soluble impurities, which is essential to achieve the required degree of purity. Filtration plays important role in the development of uniform crystalline form, since externally introduced nuclei of crystallization can sometimes be removed only this way. Subsequent to filtration, the filtrate is cooled, which results in the crystallization of the dissolved substance. In order to enhance crystallization, the suspension is cooled further with ice water or another coolant and the resulting crystalline product is filtered.

In the International Patent Application No. WO 01/28999, a process for the preparation of amorphous atorvastatin hemicalcium has been disclosed. Said process comprises precipitation of atorvastatin hemicalcium from a protic solvent, for example, from 2- propanol. During the industrial application of the process, the objective of the inventors was to achieve better yield. According to the general experience in similar cases, increasing the amount of the less polar or aprotic solvent results in increasing yield. The greatest applicable amount of the aprotic solvent is limited by the purity requirements of the product. Furthermore, when atorvastatin hemicalcium salt was precipitated as an amorphous solid from protic solvents, one skilled in the art would expect that using a mixture of a protic solvent and a less polar aprotic solvent will provide favourable conditions for the formation of an amorphous product.

Similar conclusion was reached by the inventors of European Patent Application No. 1 237 864, who dissolved atorvastatin hemicalcium in the protic solvent methanol, concentrated the solution by partly evaporating the solvent and mixed the resulting solution with diethylether, which resulted in the formation of amorphous atorvastatin hemicalcium salt.

### SUMMARY OF THE INVENTION

The technical problem to be solved by our research was to provide a process for the preparation of a crystalline atorvastatin hemicalcium salt in a high purity, stable, uniform, non-hygroscopic form suitable for the use in the preparation of medicinal products.

The above objective has been solved according to the present invention.

Surprisingly, we have found that by dissolving crude, amorphous or crystalline atorvastatin hemicalcium salt, solvates or mixtures thereof in a protic solvent or in a mixture comprising one or more protic solvent and an aprotic solvent, optionally by heating with subsequent filtering or optional incubation at a constant temperature, addition of a seeding crystal, a new crystalline polymorph form of atorvastatin hemicalcium salt can be obtained upon cooling, said solvent being:
a mixture of methanol and hexane, or
a mixture of methanol and diisopropyl ether, or a mixture of methanol and acetone

### DETAILED DESCRIPTION OF THE INVENTION

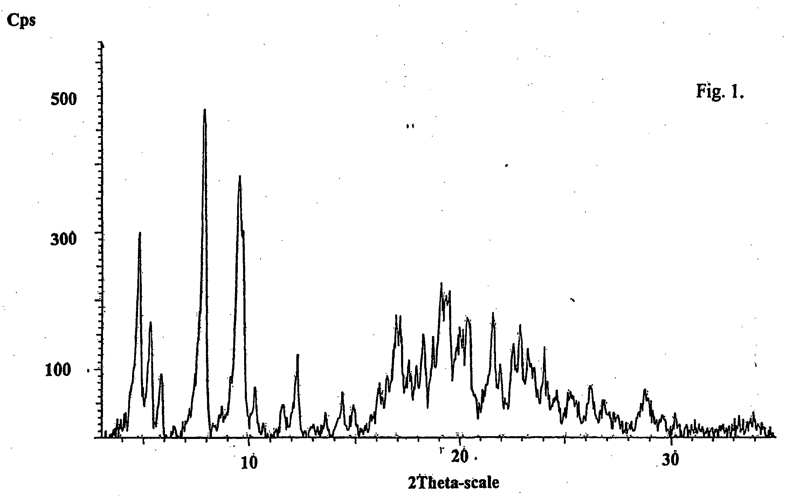

There is disclosed a new crystalline polymorph B-52 form of atorvastatin hemicalcium salt [(βR, δR)-2-(4-fluorophenyl)-β, δ-dihydroxy-5-(1-methylethyl)-3-phenyl-4-{(phenylamino)-carbonyl}-1H-pyrrole-1-heptanoic acid hemicalcium salt] of the Formula (I), which correspond to the X-ray diffraction data demonstrated in Table 1 and in Figure 1.

Crystalline polymorph form B-52 of atorvastatin hemicalcium is a high purity, stable, non-hygroscopic substance, suitable for the use in the preparation of medicinal products, having advantageous properties for the use in pharmaceutical technology.

X-ray diffraction data of the crystalline form B-52 atorvastatin hemicalcium polymorph form have been determined under the following experimental conditions.

| | |
|---|---|
| Instrument: | Bruker D8 Advanced Powder Diffractometer |
| Radiation: | CuK_{α1} (λ=1,54060 Å), CuK_{α2} (λ=1,54439 Å) |
| Voltage | 40 kV |
| Plate current | 30 mA |
| Accessories | Göbel mirror |
| | Soller slit |

During the determination of X-ray diffraction data, the reference substance SRM 640c (Silicon Powder Lot. No. H-375) was used. Measurements were carried out continously in the diffraction angle range of 4 to 30° (2Θ) in 0.04° steps. The X-ray analysis was carried out on smooth samples stored at room temperature without milling. Measurements were made at room temperature.
X-ray diffraction pattern of the atorvastatin hemicalcium polymorph B-52 form is demonstrated in Figure 1. Data of the X-ray diffraction signals are listed in Table 1.

Data of Figure 1 and Table 1 are to be interpreted in the present application by taking into account the repeatability of X-ray diffraction measurements known according to the state of the art. It is known from the state of the art, for example, from pharmacopoeias that the repeatability of the measurement of X-ray diffraction angles is approximately ±0,2°. Furthermore it is known according to the state of the art that the intensity of the X-ray diffraction peaks is significantly influenced by the sample condition or the sample preparation method. In the present patent application, polymorph forms of atorvastatin hemicalcium salt are considered identical if their measured X-ray diffraction angles correspond to each other within the above mentioned repeatability and the relative intensities determined at a given diffraction angle do not differ more than about ±20 relative percent.

According to the present invention, there is provided a process for the preparation of atorvastatin hemicalcium salt of the Formula (I) in the B-52 polymorph form, which comprises dissolving crude, amorphous or crystalline atorvastatin hemicalcium salt or solvates or mixtures thereof in a protic solvent or in a protic solvent mixture, which also contains an aprotic solvent said solvent being:
- a mixture of methanol and hexane, or
- a mixture of methanol and diisopropyl ether, or
- a mixture of methanol and acetone filtering the solution, seeding the solution with crystalline B-52 form atorvastatin hemicalcium salt seeding crystals, cooling and stirring the mixture at room temperature and filtering, washing and drying the crystals of the atorvastatin hemicalcium polymorph B-52 form thus obtained.

The weight of the solvent or the solvent mixture used can be chosen according to the properties of the solvent or the solvent mixture between 2-fold and 50-fold weight of the starting substance.

If necessary, heating is applied to facilitate the dissolution of the starting atorvastatin hemicalcium salt.

After filtering the solution containing atorvastatin calcium, the mixture is cooled to room temperature, seeding crystals are added and the mixture is stirred at room temperature for 0.1 to 48 hours, preferably, for 4 to 20 hours. The crystals of atorvastatin hemicalcium salt polymorph B-52 form thus obtained are filtered off, washed and dried.

**Table 1**

| X-ray diffraction data of atorvastatin hemicalcium salt polymorph B-52 form | | | |
|---|---|---|---|
| **Diffraction Angle (2Θ) (°)** | **d value (Å)** | **Intensity (cps)** | **Relative Intensity (%)** |
| 4,485 | 19,6865 | 105 | 21,7 |
| 4,715 | 18,7267 | 298 | 61,9 |
| 5,270 | 16,7560 | 168 | 34,9 |
| 5,766 | 15,3161 | 91,6 | 19,0 |
| 7,806 | 11,3165 | 481 | 100 |
| 9,535 | 9,26799 | 383 | 79,7 |
| 10,245 | 8,62703 | 71,9 | 14,9 |
| 11,582 | 7,63438 | 46,4 | 9,6 |
| 12,177 | 7,26235 | 88,5 | 18,4 |
| 14,327 | 6,17731 | 56,5 | 11,7 |
| 16,097 | 5,50159 | 78,7 | 16,3 |
| 16,480 | 5,37470 | 87,6 | 18,2 |
| 16,911 | 5,23878 | 178 | 36,9 |
| 17,083 | 5,18634 | 163 | 33,8 |
| 17,542 | 5,05155 | 108 | 22,5 |
| 17,887 | 4,95502 | 104 | 21,6 |
| 18,243 | 4,85894 | 150 | 31,1 |
| 18,691 | 4,74367 | 144 | 30,0 |
| 19,093 | 4,64470 | 225 | 46,7 |
| 19,437 | 4,56314 | 212 | 44,1 |
| 19,983 | 4,43979 | 160 | 33,3 |
| 20,356 | 4,35922 | 172 | 35,7 |
| 21,562 | 4,11808 | 181 | 37,5 |
| 21,935 | 4,04884 | 101 | 21,1 |
| 22,595 | 3,93198 | 131 | 27,3 |
| 22,887 | 3,88247 | 163 | 33,9 |
| 23,284 | 3,81716 | 126 | 26,1 |
| 24,085 | 3,69203 | 130 | 27,1 |
| 24,597 | 3,61630 | 67,1 | 13,9 |
| 25,297 | 3,51784 | 66,0 | 13,7 |
| 26,224 | 3,39554 | 74,2 | 15,4 |
| 26,846 | 31,31830 | 56,6 | 11,8 |
| 28,797 | 3,09776 | 66,9 | 13,9 |

there are disclosed medicinal preparations comprising atorvastatin hemicalcium salt polymorph B-52 form and one or more pharmaceutically acceptable vehicle or auxiliary agent.

The medicinal preparations generally contain 0.1-95 weight%, advantageously 1-50 weight%, the most preferably 5-30 weight% active ingredient.

The medicinal preparations can be administered orally (for example, in the form of powders, tablets, coated tablets, capsules, microcapsules, dragees, solutions, emulsions etc.), parenterally (for example, as intravenous, intramuscular, subcutaneous or intraperitoneal injection solutions or as solution for infusion), rectally (e.g. in the form of suppositories), transdermally (e.g. as patches), as implants or topically (e.g. creams, ointments or patches). The solid, semisolid, soft or liquid medicinal preparation can be prepared according to processes known for the person skilled in the art.

Solid medicinal preparations suitable for oral administration containing the polymorph B-52 form of atorvastatin hemicalcium salt can contain vehicles or fillers (e.g. lactose, lactose monohydrate, glucose, starch, calcium phosphate, calcium carbonate, microcrystalline cellulose), binders (e.g. gelatine, sorbite, polyvinyl-pyrrollidone), disintegrants (e.g. croscarmellose, sodium carboxymethyl cellulose, crospovidone) tabletting aids (e.g. magnesium stearate, talc, polyethylene glycol, silica, silicon dioxide), pH adjusting auxiliary agents (e.g. citric acid, phosphoric acid, lactic acid and alkali metal or alkali earth metal salts thereof; alkali metal or alkali earth metal carbonates or hydrogencarbonates, amino acids or amino-derivatives of carbohydrates) or surfactants (e.g. sodium laurylsulphate). Solid medicinal preparations can contain coating agents, e.g. hydroxypropylcellulose, hydroxypropyl-methylcellulose, polyvinylalcohol, polyethylene glycol, acrylate polymers, titanium dioxide or iron-oxide.

Liquid pharmaceutical preparations containing atorvastatin hemicalcium salt in the polymorph B-52 form suitable for oral administration can be solutions, suspensions or emulsions and can contain suspending agents (e.g. gelatine, carboxymethylcellulose), emulsifying agents (e.g. sorbitane monooleate), solvents (e.g. water, oils, glycerol, propylene glycol, ethanol), pH adjusting agents (e.g. acetate, phosphate, citrate buffers) and stabilizing agents (e.g. methyl-4-hydroxy-benzoate).

Liquid pharmaceutical preparations containing atorvastatin hemicalcium salt polymorph B-52 form are usually sterile isotonic solutions, which contain besides the solvent pH adjusting and conserving agents.

Soft medicinal preparations containing atorvastatin hemicalcium polymorph B-52 form as active ingredient, e.g. suppositories contain said active ingredient homogeneously dispersed in the vehicle of said preparation (e.g. polyethylene glycol, cocoa butter).

Medicinal preparations containing the atorvastatin hemicalcium polymorph B-52 form as active ingredient can be prepared according to methods of pharmaceutical technology known from the state of the art. The active ingredient is mixed with solid or liquid vehicles and auxiliary agents and the mixture is brought to galenic form. Vehicles and auxiliary agents suitable for use in medicinal products have been disclosed in the literature (Remington's Pharmaceutical Sciences, Edition 18, Mack Publishing Co., Easton, USA, 1990).

Medicinal preparations contain atorvastatin hemicalcium polymorph B-52 form in a dosage unit form.

It is disclosed the use of crystalline atorvastatin hemicalcium B-52 polymorf form for the manufacture of medicinal preparations suitable for the reduce elevated plasma cholesterol, low density lipoprotein cholesterol, apo-β-lipoprotein- or triglyceride concentration and the treatment of hypercholesterolemia, dysbetalipoproteinemia and dyslipidemia, which comprises mixing crystalline atorvastatin hemicalcium salt B-52 polymorph form with pharmaceutically suitable vehicles and auxiliary agents and bringing the mixture to galenic form.

there is disclosed a method for the reduction of elevated plasma cholesterol, low density lipoprotein cholesterol, apo-β-lipoprotein- and triglyceride level and treatment of hypercholesterolemia, dysbetalipoproteinemia and dyslipidemia in a patient in need of such treatment, which comprises administering said patient an effective dose of crystalline atorvastatin hemicalcium salt B-52 polymorph form.

Further details of the present invention are provided in the following examples without limiting the scope of protection to said examples.

### Example 1

### Preparation of crystalline (3R, 5R)-3,5-dihydroxy-[3-phenyl-4-[(phenylamino)-carbonyl]-2-(4-(fuorophenyl)-5-(1-methylethyl)-1H-pyrrole-1-yl]-heptanoic acid hemicalcium salt B-52 polymorph form from crude atorvastatin hemicalcium salt

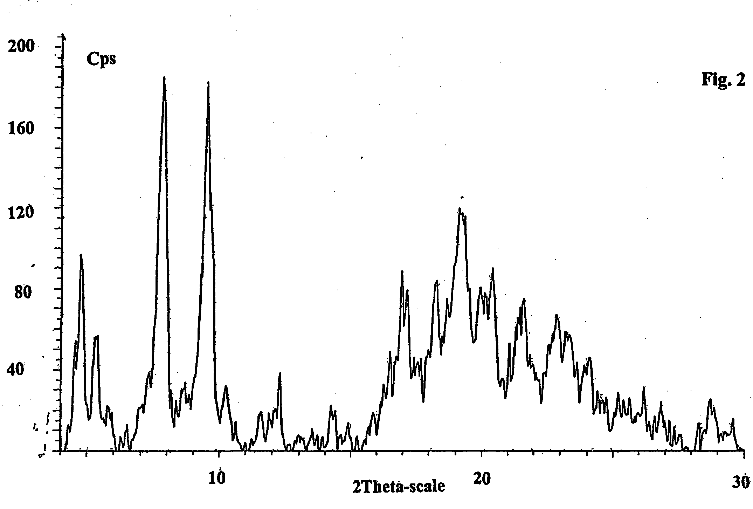

17 g crude atorvastatin hemicalcium salt prepared according to the method disclosed in European Patent No. 409281 are dissolved in the mixture of 100 ml of methanol and 150 ml of hexane at the boiling temperature of the mixture, until a clear solution is obtained. The solution is filtered, cooled to room temperature and subsequently stirred at room temperature for 12 hours. The crystals thus produced are filtered, washed with hexane and diethylether and dried. Yield 13,7 g (83%).
Results obtained by the X-ray diffraction analysis of the product are listed in Table 2. X-ray diffractogram of the product is shown in Figure 2.

**Table 2**

| **Diffraction Angle (2Θ) (°)** | **d value (Å)** | **Intensity (cps)** | **Relative Intensity (%)** |
|---|---|---|---|
| 4,50 | 19,62575 | 52,6 | 28,4 |
| 4,70 | 18,81665 | 96,2 | 51,9 |
| 5,28 | 16,72994 | 56,4 | 30,4 |
| 5,75 | 15,35783 | 23,0 | 12,4 |
| 7,79 | 11,34293 | 185 | 100 |
| 9,50 | 9,30041 | 183 | 98,9 |
| 10,24 | 8,63159 | 31,6 | 17,1 |
| 11,52 | 7,67524 | 19,1 | 10,3 |
| 12,23 | 7,22997 | 38,2 | 20,6 |
| 14,23 | 6,21810 | 22,4 | 12,1 |
| 16,48 | 5,37343 | 49,7 | 26,8 |
| 16,90 | 5,24088 | 87,7 | 47,3 |
| 17,12 | 5,17615 | 79,9 | 43,1 |
| 18,23 | 4,86192 | 83,9 | 45,3 |
| 19,18 | 4,62341 | 118 | 63,7 |
| 19,91 | 4,45670 | 79,2 | 42,8 |
| 20,33 | 4,36386 | 84,3 | 45,5 |
| 21,49 | 4,13189 | 71,5 | 38,6 |
| 22,83 | 3,89231 | 67,0 | 36,1 |
| 23,27 | 3,82014 | 58,7 | 31,7 |
| 24,09 | 3,69128 | 45,4 | 24,5 |
| 28,72 | 3,10609 | 24,8 | 13,4 |

### Example 2

### Preparation of crystalline (3R, 5R)-3,5-dihydroxy-[3-phenyl-4-[(phenylamino)-carbonyl]-2-(4-fluorophenyl)-5-(1-methylethyl)-1H-pyrrole-1-yl]-heptanoic acid hemicalcium salt B-52 polymorph form from amorphous atorvastatin hemicalcium salt

17 g amorphous atorvastatin hemicalcium salt prepared according to the disclosure of European Patent No. 1 235 799 is stirred until dissolution in the mixture of 100 ml of methanol and 150 ml of hexane at the boiling temperature of the mixture. Thus, a clear solution is obtained, which is filtered and cooled to room temperature and stirred at room temperature for further 12 hours. The precipitated crystals are filtered off, washed with hexane and diethylether and dried. Yield 13,7 g (83%).

### Example 3

### Preparation of crystalline (3R, 5R)-3,5-dihydroxy-[3-phenyl-4-[(phenylamino)-carbonyl]-2-(4-ffuorophenyl)-5-(1-methylethyl)-1H-pyrrole-1-yl]-heptanoic acid hemicalcium salt B-52 polymorph form from crude atorvastatin hemicalcium salt

17 g of crude atorvastatin hemicalcium salt prepared according to the disclosure of European Patent No. 409281 are dissolved in the mixture of 100 ml of methanol and 170 ml of diisopropylether at boiling temperature of the mixture, until a clear solution is obtained. The solution is filtered, cooled to room temperature and stirred at room temperature for further 12 hours. The crystals thus obtained were washed with hexane and diethylether and dried. Yield, 14,5 g (85%).

### Example 4 (not according to the invention)

### Preparation of crystalline (3R, 5R)-3,5-dihydroxy-[3-phenyl-4-[(phenylamino)-carbonyl]-2-(4-fluorophenyl)-5-(1-methylethyl)-1H-pyrrole-1-yl]-heptanoic acid hemicalcium salt B-52 polymorph form from crude atorvastatin hemicalcium salt

17 g of crude atorvastatin hemicalcium salt prepared according to the disclosure of European Patent No. 409281 was added to the mixture of 100 ml of methanol and 2 ml of water, heated to 40 °C, and stirred for further 30 minutes at the same temperature. The mixture is filtered while hot and the filtrate is mixed with 1 g crystalline atorvastatin hemicalcium salt polymorph B-52 form. The obtained mixture is stirred at room temperature for 12 hours. The crystalline solids are filtered off, washed with methanol and dried. Yield, 13,6 g (80%).

### Example 5 (not according to the invention)

### Preparation of crystalline (3R, 5R)-3,5-dihydroxy-[3-phenyl-4-[(phenylamino)-carbonyl]-2-(4-fluorophenyl)-5-(1-methylethyl)-1H-pyrrole-1-yl]-heptanoic acid hemicalcium salt B-52 polymorph form from crude atorvastatin hemicalcium salt

17 g of crude atorvastatin hemicalcium salt obtained according to the processes described in European Patent No. 409281 are added to 100 ml methanol, the mixture is heated to boiling temperature and stirred at the same temperature for 30 minutes. The mixture is filtered while hot and the filtrate is mixed with 1 g of crystalline atorvastatin hemicalcium salt polymorph B-52 form. Subsequently, the mixture is stirred at room temperature for 12 hours. The crystalline solids thus obtained are filtered off, washed with methanol and dried. Yield, 13,6 g (80%). The X-ray diffractogram of the product is shown in Figure 1. X-ray diffraction data of the product are given in Table 1.

### Example 6 (not according to the invention)

### Preparation of crystalline (3R, 5R)-3,5-dihydroxy-[3-phenyl-4-[(phenylamino)-carbonyl]-2-(4-fluorophenyl)-5-(1-methylethyl)-1H-pyrrole-1-yl]-heptanoic acid hemicalcium salt B-52 polymorph form from crystalline atorvastatin hemicalcium salt polymorph I form

17 g of atorvastatin hemicalcium salt polymorph I form prepared according to the process disclosed in European Patent No. 848705 are added to 100 ml of methanol, the mixture is heated to boiling and stirred at boiling temperature for 30 minutes. The mixture is filtered while hot and the filtrate are added to 1 g crystalline atorvastatin hemicalcium salt polymorph B-52 form.
The mixture is subsequently stirred at room temperature for 12 hours. The crystalline solid product is filtered off, washed with methanol and dried. Yield, 13,6 g (80%).

### Example 7

### Preparation of crystalline (3R, 5R)-3,5-dihydroxy-[3-phenyl-4-[(phenylamino)-carbonyl]-2-(4-fluorophenyl)-5-(1-methylethyl)-1H-pyrrole-1-yl]-heptanoic acid hemicalcium salt B-52 polymorph form from crystalline atorvastatin hemicalcium salt polymorph I form

17 g of crude atorvastatin hemicalcium salt prepared according to the process disclosed in European Patent No. 409281 are added to the mixture of 50 ml of methanol and 125 ml of acetone, the mixture is heating to boiling and stirred at boiling temperature for further 30 minutes. The mixture is filtered while hot and 1 g crystalline atorvastatin hemicalcium salt polymorph B-52 form are added to the filtrate. The mixture is stirred at room temperature for further 12 hours. The crystalline solids are filtered off, washed with methanol and dried. Yield, 12,6 g (74%).

## Claims

1. Process for the preparation of crystalline atorvastatin hemicalcium salt [(βR, δR)-2-(4-fluorophenyl)-β, δ-dihydroxy-5-(1-methylethyl)-3-phenyl-4-{(phenylamino)-carbonyl}-1H-pyrrole-1-heptanoic acid hemicalcium salt] polymorph B-52 form having an X-ray diffractogram essentially identical to that depicted in Figure 1 and which is **characterized by** the following X-ray diffraction data:
| Diffraction Angle (2θ) (°) | d value (Å) | Intensity (cps) | Relative intensity (%) |
|---|---|---|---|
| 4,485 | 19,6865 | 105 | 21,7 |
| 4,715 | 18,7267 | 298 | 61,9 |
| 5,270 | 16,7560 | 168 | 34,9 |
| 5,766 | 15,3161 | 91,6 | 19,0 |
| 7,806 | 11,3165 | 481 | 100 |
| 9,535 | 9,26799 | 383 | 79,7 |
| 10,245 | 8,62703 | 71,9 | 14,9 |
| 11,582 | 7,63438 | 46,4 | 9,6 |
| 12,177 | 7,26235 | 88,5 | 18,4 |
| 14,327 | 6,17731 | 56,5 | 11,7 |
| 16,097 | 5,50159 | 78,7 | 16,3 |
| 16,480 | 5,37470 | 87,6 | 18,2 |
| 16,911 | 5,23878 | 178 | 36,9 |
| 17,083 | 5,18634 | 163 | 33,8 |
| 17,542 | 5,05155 | 108 | 22,5 |
| 17,887 | 4,95502 | 104 | 21,6 |
| 18,243 | 4,85894 | 150 | 31,1 |
| 18,691 | 4,74367 | 144 | 30,0 |
| 19,093 | 4,64470 | 225 | 46,7 |
| 19,437 | 4,56314 | 212 | 44,1 |
| 19,983 | 4,43979 | 160 | 33,3 |
| 20,356 | 4,35922 | 172 | 35,7 |
| 21,562 | 4,11808 | 181 | 37,5 |
| 21,935 | 4,04884 | 101 | 21,1 |
| 22,595 | 3,93198 | 131 | 27,3 |
| 22,887 | 3,88247 | 163 | 33,9 |
| 23,284 | 3,81716 | 126 | 26,1 |
| 24,085 | 3,69203 | 130 | 27,1 |
| 24,597 | 3,61630 | 67,1 | 13,9 |
| 25,297 | 3,51784 | 66,0 | 13,7 |
| 26,224 | 3,39554 | 74,2 | 15,4 |
| 26,846 | 3,31830 | 56,6 | 11,8 |
| 28,797 | 3,09776 | 66,9 | 13,9 |
the repeatability of the measurement of X-Ray diffraction angles being approximatively ±0,2° and of the intensities determined at a given diffraction angle being no more than ±20 relative percent, said process comprising:
- dissolving crude, amorphous or crystalline atorvastatin hemicalcium salt, solvates thereof or mixtures thereof in a protic solvent or in a protic solvent mixture which can optionally contain an aprotic solvent,
- filtering,
- seeding the clear solution with crystals of atorvastatin hemicalcium salt polymorph B-52 form,
- cooling the mixture to room temperature, optionally incubating said mixture at constant temperature and
- separating the crystalline atorvastatin hemicalcium salt polymorph B-52 form, said process comprising as solvent:
- a mixture of methanol and hexane, or
- a mixture of methanol and diisopropyl ether or
- a mixture of methanol and acetone.

2. Process for preparation of crystalline atorvastatin hemicalcium polymorph B-52 form according to claim 1, which comprises:
- dissolving crude atorvastatin hemicalcium salt, or its solvate in a protic solvent or in a protic solvent mixture which can optionally contain an aprotic solvent,
- filtering,
- seeding the clear solution with crystals of atorvastatin hemicalcium salt polymorph B-52 form,
- cooling the mixture to room temperature, optionally incubating said mixture at constant temperature and
- separating the crystalline atorvastatin hemicalcium salt polymorph B-52 form.

3. Process for preparation of crystalline atorvastatin hemicalcium polymorph B-52 form according to claim 1, which comprises:
- dissolving amorphous atorvastatin hemicalcium salt or its solvate in a protic solvent or in a protic solvent mixture which can optionally contain an aprotic solvent,
- filtering,
- seeding the clear solution with crystals of atorvastatin hemicalcium salt polymorph B-52 form,
- cooling the mixture to room temperature, optionally incubating said mixture at constant temperature and
- separating the crystalline atorvastatin hemicalcium salt polymorph B-52 form.

4. Process for preparation of crystalline atorvastatin hemicalcium polymorph B-52 form according to claim 1, which comprises :
- dissolving crystalline atorvastatin hemicalcium salt or its solvate in a protic solvent or in a protic solvent mixture,
- filtering,
- seeding the clear solution with crystals of atorvastatin hemicalcium salt polymorph B-52 form,
- cooling the mixture to room temperature, optionally incubating said mixture at constant temperature and
- separating the crystalline atorvastatin hemicalcium salt polymorph B-52 form.

## Patentansprüche

1. Prozess für die Zubereitung einer kristallinen polymorphen B-52-Form von Atorvastatin-Hemicalciumsalz [(βR,δR)-2-(4-Fluorphenyl)-β,δ-dihydroxy-5-(1-methylethyl)-3-phenyl-4-{(phenylaminocarbonyl)}-1H-pyrrol-1-heptansäure-Hemicalciumsalz] mit einem Röntgenbeugungsdiagramm, welches im Wesentlichen identisch ist zu dem, welches in Figur 1 dargestellt ist und welches durch die folgenden Röntgenbeugungsdaten gekennzeichnet ist:
| Beugungswinkel (2θ)(°) | d-Wert (Å) | Intensität (cps) | Relative Intensität (%) |
|---|---|---|---|
| 4,485 | 19,6865 | 105 | 21,7 |
| 4,715 | 18,7267 | 298 | 61,9 |
| 5,270 | 16,7560 | 168 | 34,9 |
| 5,766 | 15,3161 | 91,6 | 19,0 |
| 7.806 | 11,3165 | 481 | 100 |
| 9,535 | 9,26799 | 383 | 79,7 |
| 10,245 | 8,62703 | 71,9 | 14,9 |
| 11,582 | 7,63438 | 46,4 | 9,6 |
| 12,177 | 7,26235 | 88,5 | 18,4 |
| 14,327 | 6,17731 | 56,5 | 11,7 |
| 16,097 | 5,50159 | 78,7 | 16,3 |
| 16,480 | 5,37470 | 87,6 | 18,2 |
| 16,911 | 5,23878 | 178 | 36,9 |
| 17,083 | 5,18634 | 163 | 33,8 |
| 17,542 | 5,05155 | 108 | 22,5 |
| 17,887 | 4,95502 | 104 | 21,6 |
| 18,243 | 4,85894 | 150 | 31,1 |
| 18,691 | 4,74367 | 144 | 30,0 |
| 19,093 | 4,64470 | 225 | 46,7 |
| 19,437 | 4,56314 | 212 | 44,1 |
| 19,983 | 4,43979 | 160 | 33,3 |
| 20,356 | 4,35922 | 172 | 35,7 |
| 21,562 | 4,11808 | 181 | 37,5 |
| 21,935 | 4,04884 | 101 | 21,1 |
| 22,595 | 3,93198 | 131 | 27,3 |
| 22,887 | 3,88247 | 163 | 33,9 |
| 23,284 | 3,81716 | 126 | 26,1 |
| 24,085 | 3,69203 | 130 | 27,1 |
| 24,597 | 3,61630 | 67,1 | 13,9 |
| 25,297 | 3,51784 | 66,0 | 13,7 |
| 26,224 | 3,39554 | 74,2 | 15,4 |
| 26,846 | 3,31830 | 56,6 | 11,8 |
| 28,797 | 3,09776 | 66,9 | 13,9 |
wobei die Wiederholbarkeit der Messung der Röntgenbeugungswinkel ungefähr ±0,2° beträgt und der Intensitäten, welche bei einem gegebenen Beugungswinkel bestimmt wurden, nicht mehr als ±20 relative Prozent beträgt, wobei der Prozess Folgendes umfasst:
- Auflösen eines rohen amorphen oder kristallinen Atorvastatin-Hemicalciumsalzes, seiner Solvaten oder seiner Mischungen in einem protischen Lösemittel oder in einer protischen Lösemittelmischung, welche gegebenenfalls ein aprotisches Lösemittel enthalten kann,
- Filtrieren,
- Beimpfen der klaren Lösung mit Kristallen der polymorphen B-52-Form von Atorvastatin-Hemicalciumsalz,
- Abkühlen der Mischung auf Raumtemperatur, gegebenenfalls Bebrüten der Mischung bei konstanter Temperatur und
- Trennen der kristallinen polymorphen B-52-Form von Atorvastatin-Hemicalciumsalz, wobei der Prozess Folgendes als Lösemittel umfasst:
- eine Mischung aus Methanol und Hexan oder
- eine Mischung aus Methanol und Diisopropylether oder
- eine Mischung aus Methanol und Aceton.

2. Prozess zur Zubereitung einer kristallinen polymorphen B-52-Form von Atorvastatin-Hemicalcium nach Anspruch 1, welcher Folgendes umfasst:
- Auflösen eines rohen Atorvastatin-Hemicalciumsalzes oder seines Solvats in einem protischen Lösemittel oder in einer protischen Lösemittelmischung, welche gegebenenfalls ein aprotisches Lösemittel enthalten kann,
- Filtrieren,
- Beimpfen der klaren Lösung mit Kristallen der polymorphen B-52-Form von Atorvastatin-Hemicalciumsalz,
- Abkühlen der Mischung auf Raumtemperatur, gegebenenfalls Bebrüten der Mischung bei konstanter Temperatur und
- Trennen der kristallinen polymorphen B-52-Form von Atorvastatin-Hemicalciumsalz.

3. Prozess zur Zubereitung einer kristallinen polymorphen B-52-Form von Atorvastatin-Hemicalcium nach Anspruch 1, welcher Folgendes umfasst:
- Auflösen eines amorphen Atorvastatin-Hemicalciumsalzes oder seines Solvats in einem protischen Lösemittel oder in einer protischen Lösemittelmischung, welche gegebenenfalls ein aprotisches Lösemittel enthalten kann,
- Filtrieren,
- Beimpfen der klaren Lösung mit Kristallen der polymorphen B-52-Form von Atorvastatin-Hemicalciumsalz,
- Abkühlen der Mischung auf Raumtemperatur, gegebenenfalls Bebrüten der Mischung bei konstanter Temperatur und
- Trennen der kristallinen polymorphen B-52-Form von Atorvastatin-Hemicalciumsalz.

4. Prozess zur Zubereitung einer kristallinen polymorphen B-52-Form von Atorvastatin-Hemicalcium nach Anspruch 1, welcher Folgendes umfasst:
- Auflösen eines kristallinen Atorvastatin-Hemicalciumsalzes oder seines Solvats in einem protischen Lösemittel oder in einer protischen Lösemittelmischung,
- Filtrieren,
- Beimpfen der klaren Lösung mit Kristallen der polymorphen B-52-Form von Atorvastatin-Hemicalciumsalz,
- Abkühlen der Mischung auf Raumtemperatur, gegebenenfalls Bebrüten der Mischung bei konstanter Temperatur und
- Trennen der kristallinen polymorphen B-52-Form von Atorvastatin-Hemicalciumsalz.

## Revendications

1. Procédé pour la préparation de la forme polymorphe cristalline B-52 du sel hémicalcique d'atorvastatine [le sel hémicalcique de l'acide (βR, δR) -2- (4-fluorophényl) - β, δ- dihydroxy - 5 - (1-méthyl-éthyl) - 3-phényl -4- {(phénylamino)-carbonyl}-1H-pyrrole-1-heptanoïque] ayant un diffractogramme des rayons X essentiellement identique à celui représenté sur la figure 1 et qui est **caractérisé par** les données suivantes de diffraction des rayons X :
| Angle de diffraction (2θ) (°) | Valeur d (Å) | Intensité (cps) | Intensité relative (%) |
|---|---|---|---|
| 4,485 | 19,6865 | 105 | 21,7 |
| 4,715 | 18,7267 | 298 | 61,9 |
| 5,270 | 16,7560 | 168 | 34,9 |
| 5,766 | 15,3161 | 91,6 | 19,0 |
| 7,806 | 11,3165 | 481 | 100 |
| 9,535 | 9,26799 | 383 | 79,7 |
| 10,245 | 8,62703 | 71,9 | 14,9 |
| 11,582 | 7,63438 | 46,4 | 9,6 |
| 12,177 | 7,26235 | 88,5 | 18,4 |
| 14,327 | 6,17731 | 56,5 | 11,7 |
| 16,097 | 5,50159 | 78,7 | 16,3 |
| 16,480 | 5,37470 | 87,6 | 18,2 |
| 16,911 | 5,23878 | 178 | 36,9 |
| 17,083 | 5,18634 | 163 | 33,8 |
| 17,542 | 5,05155 | 108 | 22,5 |
| 17,887 | 4,95502 | 104 | 21,6 |
| 18,243 | 4,85894 | 150 | 31,1 |
| 18,691 | 4,74367 | 144 | 30,0 |
| 19,093 | 4,64470 | 225 | 46,7 |
| 19,437 | 4,56314 | 212 | 44,1 |
| 19,983 | 4,43979 | 160 | 33,3 |
| 20,356 | 4,35922 | 172 | 35,7 |
| 21,562 | 4,11808 | 181 | 37,5 |
| 21,935 | 4,04884 | 101 | 21,1 |
| 22,595 | 3,93198 | 131 | 27,3 |
| 22,887 | 3,88247 | 163 | 33,9 |
| 23,284 | 3,81716 | 126 | 26,1 |
| 24,085 | 3,69203 | 130 | 27,1 |
| 24,597 | 3,61630 | 67,1 | 13,9 |
| 25,297 | 3,51784 | 66,0 | 13,7 |
| 26,224 | 3,39554 | 74,2 | 15,4 |
| 26,846 | 3,31830 | 56,6 | 11,8 |
| 28,797 | 3,09776 | 66,9 | 13,9 |
la répétabilité relative de la mesure des angles de diffraction des rayons X étant approximativement ± 0,2° et celle des intensités déterminées à un angle de diffraction donné n'étant pas supérieure à ± 20%,
ledit procédé comprenant:
- la dissolution de sel hémicalcique d'atorvastatine à l'état brut, amorphe ou cristallin ou des produits de solvatation de celui-ci ou des mélanges de ceux-ci dans un solvant protique ou dans un mélange de solvants protiques qui peut éventuellement contenir un solvant aprotique,
- la filtration,
- l'ensemencement de la solution claire avec des cristaux de la forme polymorphe B-52 de sel hémicalcique de l'atorvastatine,
- le refroidissement du mélange à la température ambiante, l'incubation éventuelle dudit mélange à une température constante et
- la séparation de la forme polymorphe cristalline B-52 du sel hémicalcique d'atorvastatine, ledit procédé comprenant comme solvant :
- un mélange de méthanol et d'hexane, ou
- un mélange de méthanol et d'éther diisopropyle ou
- un mélange de méthanol et d'acétone.

2. Procédé pour la préparation de la forme polymorphe cristalline B-52 du sel hémicalcique d'atorvastatine selon la revendication 1, qui comprend:
- la dissolution de sel hémicalcique d'atorvastatine à l'état brut ou un produit de solvatation de celui-ci dans un solvant protique ou dans un mélange de solvants protiques qui peut éventuellement contenir un solvant aprotique,
- la filtration,
- l'ensemencement de la solution claire avec des cristaux de la forme polymorphe B-52 du sel hémicalcique de l'atorvastatine,
- le refroidissement du mélange à la température ambiante, l'incubation éventuelle dudit mélange à une température constante et
- la séparation de la forme polymorphe cristalline B-52 du sel hémicalcique d'atorvastatine.

3. Procédé pour la préparation de la forme polymorphe cristalline B-52 de sel hémicalcique d'atorvastatine selon la revendication 1, qui comprend:
- la dissolution de sel hémicalcique d'atorvastatine amorphe ou un produit de solvatation de celui-ci dans un solvant protique ou dans un mélange de solvants protiques qui peut éventuellement contenir un solvant aprotique,
- la filtration,
- l'ensemencement de la solution claire avec des cristaux de la forme polymorphe B-52 de sel hémicalcique de l'atorvastatine,
- le refroidissement du mélange à la température ambiante, l'incubation éventuelle dudit mélange à une température constante et
- la séparation de la forme polymorphe cristalline B-52 du sel hémicalcique d'atorvastatine.

4. Procédé pour la préparation de la forme polymorphe cristalline B-52 du sel hémicalcique d'atorvastatine selon la revendication 1, qui comprend:
- la dissolution de sel hémicalcique d'atorvastatine cristallin ou un produit de solvatation de celui-ci dans un solvant protique ou dans un mélange de solvants protiques,
- la filtration,
- l'ensemencement de la solution claire avec des cristaux de la forme polymorphe B-52 de sel hémicalcique de l'atorvastatine,
- le refroidissement du mélange à la température ambiante, l'incubation éventuelle dudit mélange à une température constante et
- la séparation de la forme polymorphe cristalline B-52 du sel hémicalcique d'atorvastatine.
